# FASCICULE DE BREVET EUROPEEN

(11) **EP 0 682 538 B1**
(45) Date de publication et mention de la délivrance du brevet: **14.05.1997**
(21) Numéro de dépôt: 94906242.6
(22) Date de dépôt: 03.02.1994
(51) Int. Cl.: A61M 5/32, A61M 5/46

(54) **DISPOSITIF ASSURANT LA PROTECTION D'UNE AIGUILLE MEDICALE ET LIMITANT LA LONGUEUR UTILE DE CELLE-CI**
VORRICHTUNG ZUM SCHUTZ VON MEDIZINISCHEN NADELN UND ZUM BEGRENZEN IHRER GEBRAUCHSLÄNGE
DEVICE FOR PROTECTING A MEDICAL NEEDLE AND LIMITING THE USEFUL LENGTH THEREOF

(30) Priorité: 03.02.1993 FR 9301153
(43) Date de publication de la demande: 22.11.1995
(73) Titulaire: M A T E F, F-92500 Rueil-Malmaison (FR)
(72) Inventeur: TRAPP, Claude, F-92500 Rueil-Malmaison (FR); CHOS, Didier, F-14000 Caen (FR)
(74) Mandataire: Hud, Robert
(86) Numéro de dépôt international: FR9400131
(87) Numéro de publication internationale: WO9417847

(56) Documents cités:
- EP-A- 0 216 460
- EP-A- 0 299 287
- WO-A-90/03814
- FR-A- 1 500 058
- FR-A- 2 624 023

## Description

La présente invention concerne les aiguilles destinées à être enfoncées dans un tissu humain, animal, végétal ou alimentaire, et principalement mais non exclusivement celles à usage médical ou vétérinaire.

Plus particulièrement, l'invention concerne un dispositif conçu pour coopérer avec une aiguille creuse à raccord normalisé d'adaptation à une seringue d'injection, mais qui peut aussi constituer un accessoire pour une aiguille d'acupuncture.

De façon connue, les aiguilles médicales sont livrées avec un embout protecteur amovible qui, avant utilisation, permet d'éviter une piqûre accidentelle ou un endommagement de l'extrémité biseautée de l'aiguille. Une fois la piqûre effectuée, il faut reprendre l'embout, le remettre en position de recouvrement de l'aiguille sans se piquer, puis jeter le tout. En outre, la profondeur de la piqûre ne dépend que de l'habileté du praticien.

Par le document FR-A-2624023 il est connu une seringue de sécurité pour prises de sang et injections équipée d'un coulisseau déplaçable le long de la seringue et qui entoure l'aiguille portée en bout de la seringue. Le coulisseau peut être déplacé longitudinalement pour découvrir, au-delà de son extrémité, une certaine longueur de l'aiguille et ce coulisseau est fendu longitudinalement entre deux lèvres dont l'une est lisse et dont l'autre porte deux creux écartés en vis-à-vis d'une saillie portée par le corps de la seringue. Ces deux creux permettent le verrouillage du coulisseau dans deux positions, c'est-à-dire une position de capotage de l'aiguille et une position dans laquelle l'extrémité de l'aiguille est découverte pour permettre une injection.

La présente invention a pour objet de remédier aux inconvénients mentionnés ci-dessus présentés par les embouts de protection connus et elle propose à cet effet un dispositif protecteur simple, efficace et peu coûteux, qui reste associé à l'aiguille lors de la piqûre, en évitant un risque de piqûre accidentelle lors de sa remise en position et en évitant le risque de l'égarer, et qui permet de règler de façon précise la longueur utile de l'aiguille pour l'opération de piqûre.

Le dispositif selon l'invention, qui est associé à une aiguille d'injection adaptable par un raccord à un moyen de préhension, est indépendant du moyen de préhension et est destiné à assurer la protection de la dite aiguille et à limiter de façon réglable la profondeur de pénétration de celle-ci. A cet effet le dispositif comporte un embout protecteur déplaçable qui entoure l'aiguille, et qui coopère par un alésage cylindrique avec un support d'aiguille cylindrique et de même diamètre, de sorte qu'il peut être déplacé longitudinalement pour découvrir au-delà de son extrémité une certaine longueur de l'aiguille, le dit embout déplaçable étant fendu longitudinalement entre deux lèvres dont l'une est lisse et dont l'autre porte plusieurs creux échelonnés en vis à vis d'une saillie portée par le support d'aiguille, et il se caractérise en ce que la saillie est de largeur plus grande que l'écartement des deux lèvres et en ce que l'embout est en un matériau élastique qui permet d'écarter une dite lèvre de l'autre lèvre pour dégager la saillie d'avec un creux.

L'invention prévoit plusieurs creux échelonnés en vis-à-vis d'une saillie, ou bien plusieurs saillies en vis-à-vis d'un creux, ce qui permet de déterminer différentes longueurs utiles pour la partie découverte de l'aiguille. Cette disposition permet au praticien de connaître avec précision la profondeur de pénétration.

Pour bien faire comprendre l'invention on en décrira ci-après, à titre d'exemple sans caractère limitatif, une forme d'exécution préférée en référence au dessin schématique annexé dans lequel :

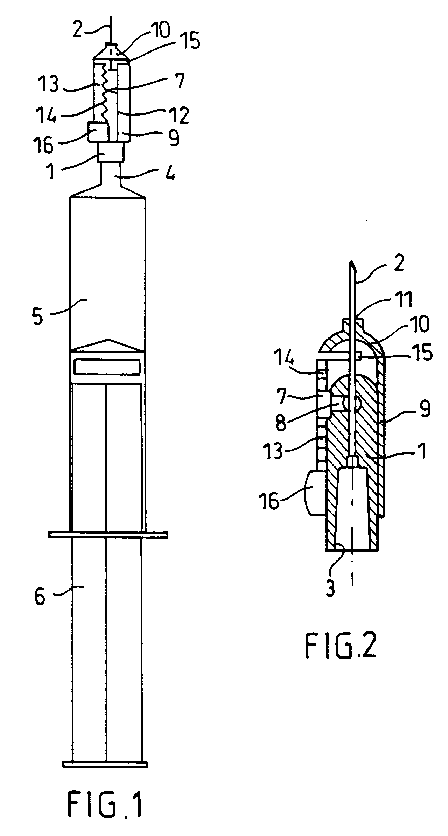

la figure 1 est une vue en plan d'une seringue d'injection portant une aiguille équipée du dispositif de protection selon l'invention ; et
la figure 2 est, à plus grande échelle, une coupe longitudinale du support de l'aiguille et de l'embout protecteur coulissant qui lui est associé.

En référence au dessin, on a représenté en 1 le support de l'aiguille 2, qui est collé sur celle-ci de façon connue et qui comporte un raccord de tout type normalisé, par exemple ici un cône creux 3 conçu pour s'adapter sur un cône mâle 4 de la seringue 5, laquelle comporte un piston 6.

Le support 1 porte une saillie 7, qui présente ici une forme de triangle et qui est solidaire d'un téton 8 fixé par collage à l'intérieur d'un forage radial du support 1. L'ensemble constitué par la saillie triangulaire 7 et le support 1 pourrait aussi être moulé d'une seule pièce.

L'embout coulissant 9, obtenu en une seule pièce moulée, présente une forme cylindrique creuse dont l'intérieur est un cylindre de même diamètre que celui du support 1, et il se termine par une extrémité protectrice 10 percée d'un trou 11 à travers lequel passe sans frottement l'aiguille 2.

La partie cylindrique de l'embout 9 est fendue longitudinalement en déterminant deux lèvres 12, 13 en regard. La lèvre 12 de la fente est lisse et s'appuie contre la face plane qui constitue la base de la pièce triangulaire 7. L'autre lèvre 13 porte des creux échelonnés tels que 14, en rrapport avec l'angle opposé de la pièce triangulaire 7 lequel constitue la saillie pénétrant dans l'un ou l'autre des creux 14.

Une fente transversale 15 coupe sensiblement à moitié l'embout 9 à proximité de son extrémité 10, ce qui permet aux lèvres 12, 13 de s'écarter élastiquement l'une de l'autre pour dégager la saillie 7 d'avec un creux 14. A sa partie inférieure la lèvre 13 présente une proéminence 16 conçue pour recevoir une poussée afin de dégager la saillie 7 d'avec un creux 14.

Le fonctionnement du dispositif selon l'invention se comprend immédiatement d'après la description qui précède.

Le dispositif étant en position de protection sur le support 1 fixé à la seringue 5, en recouvrant totalement l'aiguille 2, le praticien au moment de l'emploi appuie sur la proéminence 16 pour dégager la saillie 7 du creux 13 dans lequel elle est engagée puis déplace l'embout coulissant 9 vers la seringue 5 jusqu'à ce que la longueur voulue de d'aiguille 2 soit découverte. En relâchant la pression appliquée sur la proéminence 16, l'embout 9 se verrouille par engagement de la saillie 7 dans le creux 14 le plus voisin.

Après usage, on appuie sur la proéminence 16 puis on repousse l'embout 9 de façon à recouvrir entièrement l'aiguille 2, et on peut dès lors jeter l'ensemble éventuellement contaminé sans risque de piqûre ou de blessure.

On notera que, l'embout coulissant 9 faisant partie du support de l'aiguille, il n'y a pas de risque de le perdre et on n'a donc pas, après la piqûre, à le rechercher sur une table, voire sur le sol.

On comprendra que la description ci-dessus a été donnée a simple titre d'exemple, sans caractère limitatif, et que des adjonctions ou des modifications constructives pourraient y être apportées sans sortir du cadre de l'invention, tel qu'il resulte des revendications suivantes.

## Revendications

1. Dispositif associé à une aiguille d'injection (2) qui est adaptable par un raccord (3) à un moyen de préhension (4), ce dispositif étant indépendant du moyen de préhension (4) et destiné à assurer la protection de la dite aiguille (2) et à limiter de façon réglable la profondeur de pénétration de celle-ci, et comportant un embout protecteur (9) déplaçable qui entoure l'aiguille (2), et qui coopère par un alésage cylindrique avec un support d'aiguille (1) cylindrique et de même diamètre, de sorte qu'il peut être déplacé longitudinalement pour découvrir au-delà de son extrémité (10) une certaine longueur de l'aiguille, le dit embout déplaçable (9) étant fendu longitudinalement entre deux lèvres dont l'une (12) est lisse et dont l'autre (13) porte plusieurs creux (14) échelonnés en vis à vis d'une saillie (7) portée par le support d'aiguille (1), la dite saillie (7) étant de largeur plus grande que l'écartement des deux lèvres (12,13) et l'embout (9) étant constitué par un matériau élastique qui permet d'écarter une dite lèvre (13) de l'autre lèvre (12) pour dégager la saillie (7) d'avec un creux (14).

2. Dispositif selon la revendication 1, caractérisé en ce que les lèvres (12,13) sont limitées à proximité de l'extrémité (10) de l'embout (9) par une fente transversale (15).

3. Dispositif selon la revendication 1 ou la revendication 2, caractérisé en ce que la lèvre lisse (12) s'appuie contre une face plane de la saillie (7).

4. Dispositif selon l'une quelconques des revendications 1 à 3, caractérisé en ce que la lèvre (13) portant les creux (14) présente une proéminence (15) servant de surface d'appui pour dégager la saillie (7) du creux (14) dans lequel elle pénètre.

## Claims

1. Device associated with a hypodermic needle (2) which can be fitted by a connection (3) to a gripping means (4), the device being independent of the gripping means (4) and being intended to protect the said needle (2) and to limit its depth of penetration in an adjustable manner, the device comprising a movable protective sleeve (9) which surrounds the needle (2) and which cooperates, by means of a cylindrical hole, with a cylindrical needle-holder (1) so as to be movable longitudinally in order to uncover a certain length of the needle beyond the end (10) of the sleeve, the said movable sleeve (9) having a longitudinal slot between two lips, of which one (12) is smooth and the other (13) has several recesses (14) arranged at intervals facing a projection (7) carried by the needle-holder (1), the said projection (7) having a width greater than the spacing of the two lips (12, 13) and the sleeve (9) being made of a resilient material which allows one (13) of the said lips to be moved away from the other lip (12) in order to release the projection (7) from a recess (14).

2. Device according to Claim 1, characterized in that the lips (12, 13) are bounded by a transverse slot (15) near the end (10) of the sleeve (9).

3. Device according to Claim 1 or Claim 2, characterized in that the smooth lip (12) bears against a flat face of the projection (7).

4. Device according to any one of Claims 1 to 3, characterized in that the lip (13) having the recesses (14) has a protuberance (15) serving as a bearing surface for releasing the projection (7) from the recess (13) which it has entered.

## Patentansprüche

1. Vorrichtung, die einer Injektionsnadel (2) zugeordnet ist, welche über ein Verbindungsstück (3) an einem Greifmittel (4) angebracht werden kann, wobei diese Vorrichtung von dem Greifmittel (4) unabhängig ist und den Schutz der Nadel (2) gewährleisten sowie auf einstellbare Weise deren Eindringtiefe begrenzen soll und eine verschiebbare Schutzendkappe (9) aufweist, die die Nadel (2) umgibt und über eine zylindrische Bohrung mit einer zylindrischen Nadelhalterung (1) gleichen Durchmessers zusammenwirkt, so daß diese in Längsrichtung verschoben werden kann, um jenseits ihres Endes (10) eine bestimmte Nadellänge freizulegen, wobei die verschiebbare Endkappe (9) in Längsrichtung zwischen zwei Lippen geteilt ist, von denen eine (12) glatt ist und die andere (13) mit mehreren verteilten Vertiefungen (14) versehen ist, die einem an der Nadelhalterung (1) angeordneten Vorsprung (7) gegenüberliegen, wobei der Vorsprung (7) breiter ist als der Abstand zwischen den beiden Lippen (12, 13) und die Endkappe (9) aus einem elastischen Material hergestellt ist, das das Entfernen einer Lippe (13) von der anderen Lippe (12) zum Freilegen des Vorsprungs (7) aus einer Vertiefung (14) gestattet.

2. Vorrichtung nach Anspruch 1, dadurch gekennzeichnet, daß die Lippen (12, 13) nahe dem Ende (10) der Endkappe (9) durch einen Querschlitz (15) begrenzt sind.

3. Vorrichtung nach Anspruch 1 oder 2, dadurch gekennzeichnet, daß die glatte Lippe (12) an einer ebenen Fläche des Vorsprungs (7) anliegt.

4. Vorrichtung nach einem der Ansprüche 1 bis 3, dadurch gekennzeichnet, daß die mit Vertiefungen (14) versehene Lippe (13) einen Überstand (15) aufweist, der als Anlagefläche zum Freigeben des Vorsprungs (7) aus der Vertiefung (14), in die er eindringt, dient.
